(19) **Europäisches Patentamt**

**European Patent Office**

**Office européen des brevets**

(11) **EP 1 333 383 A1**

(12) **EUROPEAN PATENT APPLICATION**

(43) Date of publication:
06.08.2003 Bulletin 2003/32

(51) Int Cl.7: **G06F 17/12**, G06F 19/00

(21) Application number: 02394017.4

(22) Date of filing: 31.01.2002

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Hitachi, Ltd.**
**Chiyoda-ku, Tokyo 101-8010 (JP)**

(72) Inventor: **Kelly, Enda Daniel**
**Raheny, Dublin 5 (IE)**

(74) Representative: **O'Connor, Donal Henry**
**c/o Cruickshank & Co.,**
**1 Holles Street**
**Dublin 2 (IE)**

(54) **Computer based system and method to solve coupled non linear equations**

(57) The invention relates to a computerised system (1) and method for the solving of coupled non-linear equations. The invention is in particular directed towards solving non-linear equations in gene hunting studies. The invention provides a system (1) and method for removing (7) and storing equations which have reached equilibrium during computation thereby greatly reducing the computation time and complexity of computer hardware required. The system (1) and method further provides a reinsertion means (8) to retrieve the stored equation for further computation when necessary thus increasing the accuracy of the system (1) results.

Fig. 1

## Description

## Introduction

The present invention relates to a computerised system and method for the solving of coupled non-linear equations having a plurality of terms and in particular to the solution of such non-linear equations in gene hunting studies and more particularly to a system which is used to analyse linkage disequilibrium (LD), namely, deviation from random occurrence of alleles in haplotypes by using an expectation-maximisation (EM) algorithm to produce the equations.

A major problem in the solution of coupled non-linear equations is the sheer computational requirements which firstly ensure that they can only be solved by computerised methods and then secondly, even though they can be solved by computerised methods, the actual load on a computerised system is such that the computational time, even with relatively powerful computers which are becoming more and more powerful everyday, is simply enormous. For example, linkage disequilibrium analysis is a process central to the discovery of the gene or genes responsible for a particular trait, be it a disease, or a benign attribute. For the researcher to produce reliable statistics, large amounts of genetic data must be analysed, necessitating computer-based tools. However, when the data appears in the phase-unknown form, the most common case, present analysis algorithms take considerable lengths of time to analyse the data, drastically slowing the work-rate of clinicians.

Because it is almost self-evident that for all practical purposes, such computation, in practice, may only be carried out by computer, little, if any, attention has been paid to the fact that even for very computationally fast computers, the solution of such equations is time consuming and hence very expensive.

The first task of the clinician intent on the discovery of the genetic make-up of a particular trait is the gathering of DNA samples. These must be taken not only from infected individuals, but also from uninfected individuals, to serve as control samples. Cells are collected by various means including blood sample and inner cheek swabs.

The latter is preferable, as the frequent taking of blood is an irritant to the individual.
DNA must then be extracted from the nucleus of the cells. Various methods exist for this, the most accurate of which requires about a day to complete. However, less time-consuming methods exist. A common and rapid technique in veterinary genetics is to bathe the cells in sodium hydroxide, which dissolves the cell, leaving only the DNA. This isolated DNA is then placed in a Polymerase Chain Reaction (PCR) machine. On average, 96 different samples of DNA are analysed in a given PCR. The PCR is a method of amplifying specific DNA segments by exploiting certain features of DNA replication. In general, the segments are the particular haplotype of interest. Each cycle of the PCR theoretically doubles the number of segments of the chosen strand of DNA. These segments are then labelled with radioactive markers which colour-code individual nucleotides, allowing the DNA segment to be subsequently sequenced. This is done via a sequencer, which analyses the strands and observes the most predominant colour at each nucleotide locus. The nucleotide corresponding to this colour is then presumed to inhabit this locus. A sample of many pieces of the same segment is used to avoid swamping and corruption of the data from contaminants. Older methods, such as Southern blot techniques, are also still in use. These are considerably more time-consuming and open to human error than the previous technique, but are significantly less expensive. Results are stored in a database which lists the haplotypes in question of each individual. These haplotypes are provisional. In general, the above technique merely produces genotypic information on the individual, without specifying the haplotype. The clinician then must make an "educated guess" as to the composition of the haplotypes and use the computerised system of the invention to correct any errors or confirm the clinicians estimate or guess. This guess is based on previous experiences, and thus can be quite accurate for the case of infected individuals. However, the estimation of the control cases is much more difficult.

Linkage disequilibrium (LD) is defined as a deviation from random occurrence of alleles in haplotypes. Essentially, what the researcher does is he or she looks at the LD between two loci and thus haplotypes constituted of two alleles are studied. If a statistically significant deviation is detected, this could imply that the two loci are in close proximity. The core algorithm used for analysing the existing LD in phase-unknown data is the Expectation-Maximisation (EM) algorithm. Application of the EM algorithm to this problem produces a set of coupled non-linear equations, one for each of the frequencies of each of the haplotypes possible given the sample data.

As explained already, except in the most simple situations, the solution of these equations may only be found by use of computerised iteration of the equations. One of the problems is that each of the equations converges to its solution at a different rate. In the conventional method, each equation is recomputed at each iteration, regardless of whether it has reached its solution or not.

Linkage disequilibrium using the EM algorithm was originally proposed by W.G. Hill in his paper "Estimation of Linkage Disequilibrium In Randomly Mating Populations" which was published in Heredity, Volume 33, pages 229-239, in 1974, but it was not until A.P. Dempster, N.M. Laird, and D.B. Rubin's paper "Maximum likelihood from incomplete data via the EM algorithm" which appears in the Journal of the Royal Statistical Society Series B, Volume 39, pages 1-38 in 1977 that the term Expectation-Maximisation was coined. Its application to the process of linkage disequilibrium analysis was outlined by M. Slatkin and L. Excoffier in the paper 'Testing for linkage disequilibrium in genotypic data using the Expectation-Maximization algorithm" which appeared in Heredity, Volume 76, pages 377-383, in 1996. R. M. Neal and G. E. Hinton in the paper "A view of the EM algorithm that justifies incremental, sparse, and other variants", on pages 355-368 of the book Learning in Graphical Models, edited by M. I. Jordan, use the term "freezing" in connection with the EM algorithm.

Neal and Hinton, in the latter paper, for cases where the unobserved variables can take on many possible values, but only a small set of "plausible" values having non-negligible probability which, in the present case, would be, for example, given by the haplotype frequencies, propose "freezing the probabilities of the implausible values for many iterations", recomputing only the relative probabilities of the plausible values at every iteration and only at infrequent intervals recomputing all values as they consider they would not have a significant effect on the other frequencies.

As explained in more detail in the description below, even a relatively simple problem where there were two alleles at a first locus and two alleles at a second locus, there are four coupled non-linear equations which would require over four million iterations. Thus, it can be seen that anything that can be done to reduce the computational time is extremely important. The present invention is directed towards this aim.

## Statements of Invention

According to the invention there is provide a computerised system for the solving of coupled non-linear equations having a plurality of terms of the type comprising:-

an input device;

a memory;

a central processing unit (CPU) connected to the input device and the memory for carrying out iterations of the non-linear equations for varying values of each term of the equation characterised in that there is provided detection means for ascertaining when an equation has reached equilibrium after a plurality of computation iterations of the equations by the CPU;

removal means to extract the equation from the CPU when the equation has reached equilibrium and to transfer the equation to the memory for storage; and

re-insertion means to retrieve the equation after a preset further number of iterations of the equation and to transfer the equation to the CPU for further iterations of the computation thereby reducing the computational time for solving the equation.

This greatly reduces the computational time of the computer.

Ideally the removal means is arranged so that, if after a preset number of iterations of the computation subsequent to re-insertion, the equation is in equilibrium, the removal means again transfers the equation to the memory from the computation.

Preferably after re-insertion of the equation, the removal means is arranged so that if the value of the equation is in equilibrium with the value of the equation prior to removal, the equation is again transferred to the memory by the removal means for the preset further iterations.

The removal means is arranged so that more than one equation may be transferred to the memory at any one time.

Further the re-insertion means are arranged so that the equation is re-inserted after between two and ten iterations and preferably this is after five iterations.

The detection means is adapted to determine that equilibrium is reached when it senses that the value of the equation for successive iterations varies by less than a preset percentage of the value of one or other of the two values which percentage may be between $10^{-2}$ and $10^{-4}$.

In another embodiment of the invention the detection means is arranged so that when a preset number of equations have reached equilibrium, the values of the equations are inserted into a likelihood equation and termination means are provided for stopping further iterations of the equations if the result of the likelihood equation is such as to indicate that further iterations are unlikely to produce a result, the likelihood of which would be possible.

In a still further embodiment in which, when at least some of the equations have reached equilibrium, the detection means is arranged to insert the values into a likelihood equation and the results of the likelihood equation are transferred to the memory. This latter operation may be carried out when all the equations have reached equilibrium.

In one embodiment of the invention in a system when conducting a gene hunting study the sum of the value of the equations equals one and the equations are deemed to be in equilibrium when the value of the equations for successive iteration varies by less than $10^{-6}$.

The system may be used to analyse linkage disequilibrium (LD) namely deviation from random occurrence of alleles in haplotypes by using an expectation-maximisation (EM) algorithm to produce the equations and the system includes means to assign values for the haplotype frequencies of the haplotypes of two alleles one from each of two loci.

In this latter embodiment the means to assign values includes means to assign values to two loci and may include means to assign random values, the aggregate of which is one.

The invention may comprise a computer program comprising program instructions which, when loaded into a computer, comprise any of the aforesaid means which computer program may be embodied on a record medium, in a computer memory, in a read-only memory or carried on an electrical signal carrier.

## Detailed Description of the Invention

The invention will be more clearly understood from the following description of some embodiments thereof, given by way of example only, with reference to the accompanying drawings, in which:-

Fig. 1 is a schematic view of a computer system according to the invention,

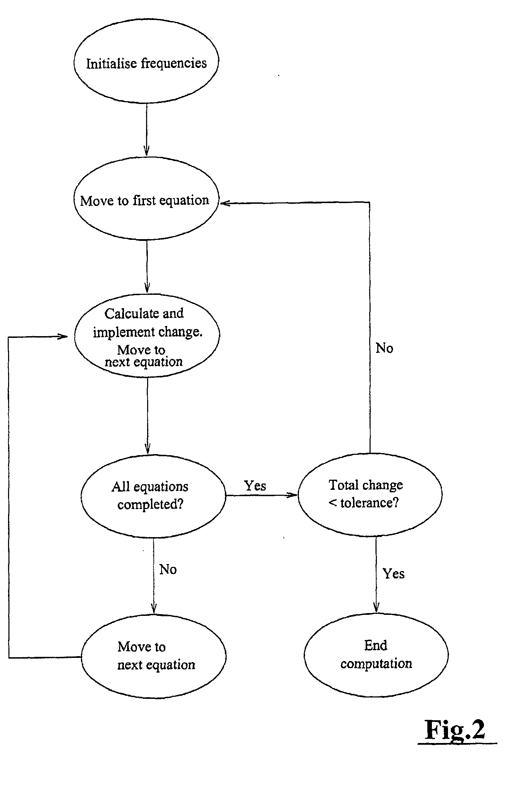

Fig. 2 is a flowchart of a conventional process,

Fig. 3 is a schematic representation of the invention, and

Fig. 4 is a flowchart of the process of the invention.

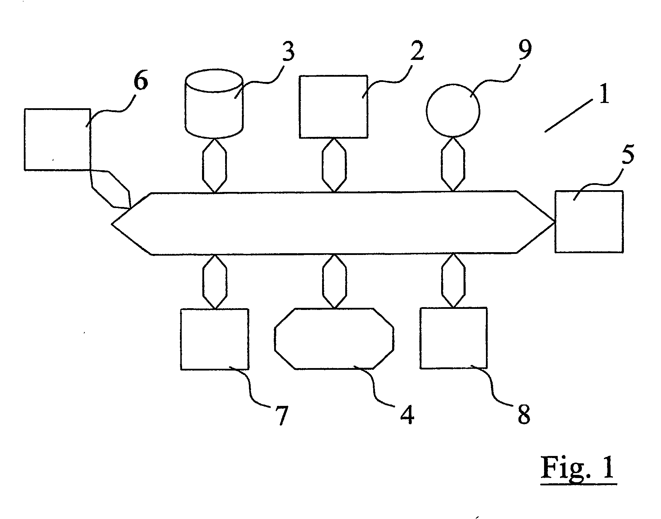

Referring to Fig. 1, there is illustrated a computerised system, indicated generally by the reference numeral 1, comprising an input device 2, a memory 3, a central processing unit (CPU) 4 and a display 5. There are provided other specific programs to provide detection means, removal means and re-insertion means whose functions will be described later. These, it will be appreciated, will be stored in RAM but are shown separately for illustrative purposes and are identified in Fig. 1 as detection means 6, removal means 7 and re-insertion means 8. Further, termination means 9 is provided to stop further computation in various circumstances. Similarly, various other means may be provided for various control functions such as means to assign values for the haplotype frequencies of the haplotypes of alleles or indeed means to assign values to various loci for gene studies and means to detect various situations that may arise. Further, various programs are provided for the system which may allow various operations to take place.

Although, in the above description, reference has been made to drawings which effectively comprise computers and various apparatus associated therewith and further to processes and methods performed in a computer, with reference to various means, the invention also extends to computer programs, particularly computer programs on or in a carrier, adapted for putting the invention into practice. As will be appreciated by those skilled in the art, such a computer program may be in the form of source code, object code, a code intermediate source and an object code such as in partially compiled form, or in any other form suitable for use in the implementation of the present invention. The carrier, needless to say, can be any device or entity capable of carrying the program and may, for example, comprise a storage medium such as a ROM, for example, a CD ROM or a SEMI-CONDUCTOR ROM or a magnetic recording medium, for example, a floppy disk or a hard disk. Further, the carrier may be a transmittable carrier such as an electrical or optical signal which may be conveyed via electrical or optical cable or by radio or other means. Indeed, it is envisaged that certain aspects of the invention may be carried out remotely from other aspects.

It will be appreciated that when the program is embodied in a signal which may be conveyed directly by a cable or other device or means, the carrier maybe constituted by such a cable or other device or means. Alternatively, the carrier may be an integrated circuit in which the program is embedded, the integrated circuit being adaptable for performing or for use in the performance of, the relevant functions carried out by the various means in accordance with the invention.

The following is the generic equation for an Expectation-Maximisation (EM) algorithm.

$$\hat{f}_{ij} = \left( \sum_{i \geq i} n_{ii'jj} + \sum_{i' < i} n_{i'ijj} + \sum_{j \geq j} n_{iijj'} + \sum_{j' < j} n_{iij'j} + \sum_{i' \neq i, j' \neq j} \frac{f_{ij} f_{i'j'}}{f_{ij} f_{i'j'} + f_{ij'} f_{i'j}} n_{abcd} \right) / 2n$$

where a = min(i,i'), b = max (i,i'), c = min (j,j'), d = max(j,j')

The equation when applied to gene hunting studies is discussed in more detail below.

When dealing with phase-unknown data, LD between a pair of loci is investigated using a likelihood-ratio test, whose empirical distribution is obtained by a permutation procedure. There are two hypothesis. The first that no LD exists (i.e. linkage equilibrium), denoted $H_0$, and the second that LD exits, denoted $H_1$. The likelihood of the data assuming linkage equilibrium ($L_{H0}$) is computed by using the fact that, under this hypothesis, the haplotype frequencies, denoted $f_{ij}$, are obtained as the product of the observed allele frequencies, i.e. say the allele frequencies at the first locus are given by $p_i$, and at the second locus by $q_j$, then assuming $H_0$, $f_{ij} = p_i q_j$. The values of $p_i$ and $q_j$ are derived from the sample data. The likelihood of the data assuming linkage disequilibrium ($L_{H1}$) is obtained by applying the Expectation-Maximisation (EM) algorithm to estimate haplotype frequencies.

The observed patient data for a particular pair of loci is represented in a contingency table. Each table holds the number of occurrences of all possible allele quadruplets for a pair of loci. An example table is illustrated in Table 1.

Table 1:

| Table for two loci with 3 alleles each | | | | | | | |
|---|---|---|---|---|---|---|---|
| | $A_1A_1$ | 5 | 16 | 3 | 0 | 7 | 9 |
| | $A_1A_2$ | 3 | 14 | 1 | 10 | 0 | 2 |
| Locus 1 | $A_1A_3$ | 1 | 26 | 3 | 4 | 12 | 4 |
| | $A_2A_2$ | 0 | 5 | 11 | 22 | 2 | 0 |
| | $A_2A_3$ | 1 | 13 | 1 | 21 | 7 | 8 |
| | $A_3A_3$ | 8 | 14 | 3 | 2 | 8 | 0 |
| | | $B_1B_1$ | $B_1B_2$ | $B_1B_3$ | $B_2B_2$ | $B_2B_3$ | $B_3B_3$ |

Referring to the table, Locus 1 has 3 alleles $A_1$, $A_2$, $A_3$. Thus, six different allele pairings are possible at this locus as there are two alleles at each locus, as illustrated by the left-hand side of the table. Similarly locus 2 also has three alleles $B_1$, $B_2$, $B_3$, again giving six possible allele pairings. Thus, the 5 in the top left-hand corner of the table denotes that 5 patients in the study had the alleles $A_1$ and $A_1$ at the first locus and the alleles $B_1$ and $B_1$ at the second locus, and so forth for the rest of the table entries. In total there are 36 possible quadruplets between the two loci. In the general case, where N alleles appear at the first locus and M at the second, the table will contain N(N + 1)/2 K∗ M (M + 1)/2 elements.

The estimated haplotype frequencies for each contingency table are derived via an iterative procedure. The likelihood of a particular table is given by

$$L \propto \prod_{ii'jj'} \left( c^{(1)}_{ii'jj'} f_{ij} f_{i'j'} + c^{(2)}_{ii'jj'} f_{i'j} f_{ij'} \right)^{n_{ii'jj'}}$$

where $c^{(1)}_{ii'jj'} = 1$, $c^{(2)}_{ii'jj'} = 0$ if $i = i'$ and $j = j'$, $c^{(1)}_{ii'jj'} = 2$, $c^{(2)}_{ii'jj'} = 2$ if $i \neq i'$ and $j \neq j'$, and $c^{(1)}_{ii'jj'} = 2$, $c^{(2)}_{ii'jj'} = 0$ otherwise.

The equilibrium solution represents a local, but not necessarily global, maximum of L, thus numerous initial guesses, usually ten, for the $f_{ij}$'s are tried, and the largest value of L retained.

The likelihood ratio statistic is given by

$$S = -2\log\left(\frac{L_{H_0}}{L_{H_1}}\right)$$

S should in principle follow a $\chi^2$ distribution with (N - 1) (M - 1) degrees of freedom, where, as before, N and M are the number of alleles at locus 1 and 2, respectively, but this is not always the case in small samples with large numbers of alleles per locus. In order to better approximate the underlying distribution of the likelihood-ratio statistics under the null hypothesis of linkage equilibrium, the following permutation procedure is used:

1. Randomly permute the alleles between individuals at one locus only to produce a new contingency table.

2. Re-estimate the likelihood of the new table $L_{H1}$ by the EM algorithm. Note that $L_{H0}$ is unaffected by the permutation procedure.

3. Repeat the steps 1-2 a large number of times to get the null distribution of $L_{H1}$ and therefore the null distribution of S.

The P-value of the test (the statistical result which is identical to the result of $\chi^2$ test) is the proportion of the visited tables which produce values of S greater than or equal to that of the observed table (i.e. we are seeing how exceptional or not the observed data is among all possible contingency tables with the same marginal values, which are given by the number of times each allele appears in the data).

This EM algorithm is programmed into the computer system 1. The user inputs the relevant parameters to the input device 2. These are stored in memory. The CPU 4 performs the operation prescribed to the software and the output device 5 delivers the result. Applied to this, the algorithm results in a set of coupled non-linear equations, one for each of the possible two-allele haplotypes that may exist between two loci. Thus, if in the sample data, there are N alleles at the first locus and M alleles at the second, then there will be N x M coupled non-linear equations to be solved. The frequency of the haplotype with the $i^{th}$ allele of the first locus and the $j^{th}$ allele of the second locus is denoted $f_{ij}$. The equations for the $f_{ij}$ are solved via an iterative procedure. Initially, the $f_{ij}$ are assigned random values. A new value for $f_{ij}$, denoted $\hat{f}_{ij}$, is calculated via the equation given above. Unless otherwise restricted by the definition of the equation, sums are from 1 to N for $i$ and $i'$ and from 1 to M for $j$ and $j$. $n_{ii'jj'}$ is the number of patients in the sample with the alleles $i$ and $i'$ at the first locus and j and $j$ at the second locus (each individual has two alleles at each locus). n is the number of patients in the sample. This is done for all N x M haplotype frequencies. At each iteration, the $\hat{f}_{ij}$ from the previous iteration become the $f_{ij}$ of the current iteration. The process is repeated until the sum of all the absolute changes to the haplotype frequencies for one iteration falls below a set tolerance. The system is then said to have converged and to be in equilibrium.

As an example, for the simplest case of N = 2 and M = 2, there are four coupled non-linear equations and these are given below.

$$\hat{f}_{00} = \left( 2n_{0000} + n_{0001} + n_{0100} + \frac{f_{00}f_{11}}{f_{00}f_{11} + f_{01}f_{10}} n_{0101} \right) / 2n$$

$$\hat{f}_{01} = \left( n_{0001} + 2n_{0011} + n_{0111} + \frac{f_{01}f_{10}}{f_{01}f_{10} + f_{00}f_{11}} n_{0101} \right) / 2n$$

$$\hat{f}_{10} = \left( n_{0100} + 2n_{1100} + n_{1101} + \frac{f_{10}f_{01}}{f_{10}f_{01} + f_{00}f_{11}} n_{0101} \right) / 2n$$

$$\hat{f}_{11} = \left( 2n_{1111} + n_{1101} + n_{0111} + \frac{f_{00}f_{11}}{f_{00}f_{11} + f_{01}f_{10}} n_{0101} \right) / 2n$$

Convergence to equilibrium of the haplotype frequencies is not uniform across the system equations. Some terms reach a stable equilibrium (i.e. they change by a negligible amount on a particular iteration) in far fewer iterations than others.

However, with the conventional method, the new $\hat{f}_{ij}$ would still be calculated for every equation until the complete system has reached equilibrium. What the present invention does is to freeze various equations that have reached equilibrium and they are not recalculated for a further fixed number of iterations of the whole system. In this present embodiment, when a term has reached equilibrium, it is then frozen for five iterations. The haplotype frequencies must sum to one in the present example. Equilibrium is said to be achieved when the value of any particular equation is such that the value of the equation for successive iterations varies by less than $10^{-6}$ between two values. Freezing is then carried on for five iterations and subsequently it is then unfrozen and inputted again and calculated again. If, however, after being unfrozen, it is in equilibrium, it is again frozen.

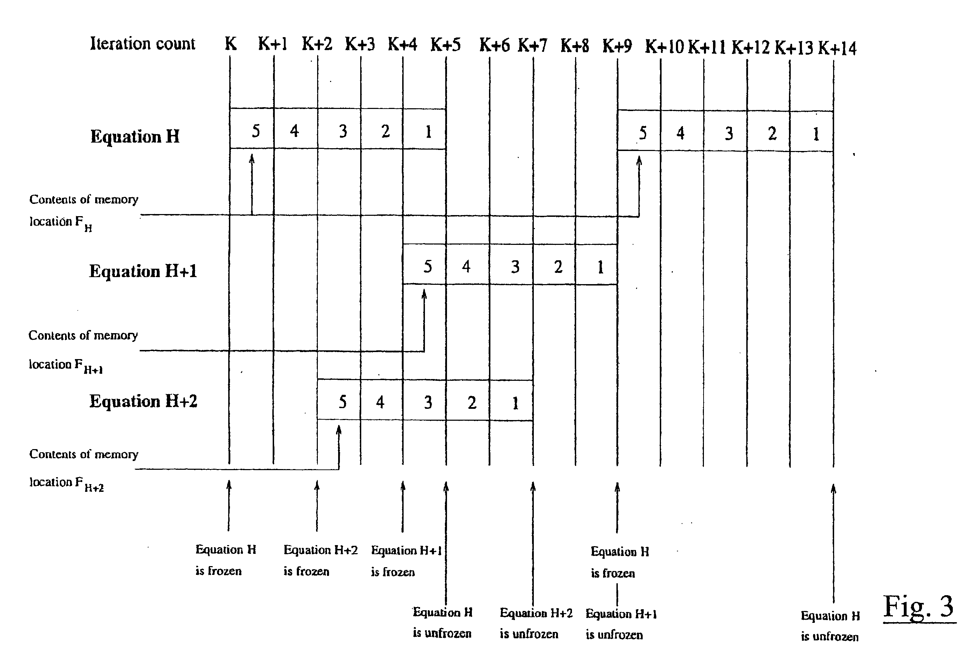

Referring now to Fig. 3, there is illustrated three equations, where various equations are frozen and unfrozen. For example, while equation H has been shown to be unfrozen for four iterations, it would be possible that it might be only unfrozen for one iteration if it's value was still in equilibrium.

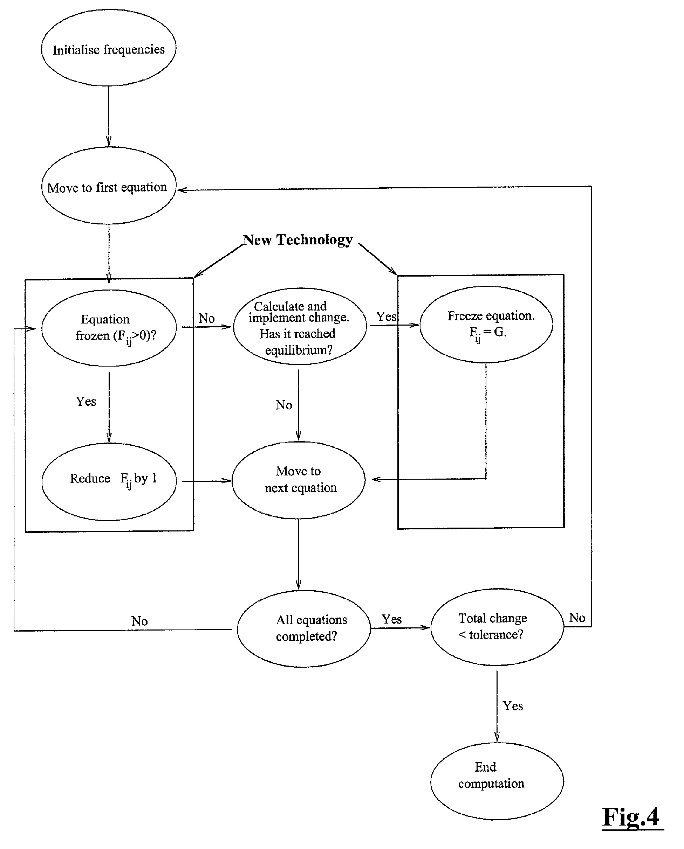

The method can be seen clearly from Fig. 4 where haplotype frequencies are not recalculated for a fixed number of iterations of the whole system. As the system is non-linear, frozen terms depend on terms which have yet to reach equilibrium. Thus, frozen terms often will be "unfrozen" and re-entered into the iterative process at some point before the iterations cease, to take into account the changes in the other frequencies. When they are not unfrozen, it can lead to a small error in the computations which is catered for by an accuracy check on the results, as described below. This check ensures that for the final values of the haplotype frequencies (when equilibrium is said to have been achieved), the average difference between the right-hand side of the nonlinear equations (4 in the simple example) and the left-hand side is below a certain tolerance (ideally the final value for $f_{ij}$ should be precisely equal to the expression on the RHS of the equation for $f_{ij}$). If not, the equations are reiterated (re-entered into the system) until the difference is below the given tolerance. Thus, freezing terms for many iterations may be counterproductive as variations within the system of haplotype frequencies while they were frozen may have shifted their equilibrium values significantly from the frozen values and thus more iterations would be required to reach equilibrium again. Thus, each haplotype frequency $f_{ij}$ is held frozen for G iterations of the system. The value of G depends on the complexity of the system and in the present example, as illustrated with reference to Fig. 3, the frequency G was set to 5. The number of iterations a particular frequency has been frozen for is kept track of by assigning a value $F_{ij}$ to each which is stored in a unique memory location for each haplotype frequency. When the frequency is first frozen $F_{ij}$ is set to G and this number is reduced by one at each iteration. When $F_{ij}$ returns to zero, the frequency is unfrozen and re-entered into the computations. This can be seen clearly in Fig. 3.

Various methods can be used to determine when equilibrium has arisen either, for example, as mentioned above, in relation to gene hunting studies, since the sum of the values in the equations must always equal one, the equation can be deemed to be in equilibrium when the value of the haplotype frequencies for successive iterations varies by less than a fixed amount such as by $10^{-6}$. Alternatively, if values can be greater than one, then essentially equilibrium is reached when the value of the unobserved variable that is sought after, namely the haplotype frequency for successive iteration varies by less than a preset percentage of the value of one or other of the two values. Whether one would use different percentages for values of different amounts is questionable and this will largely depend on the situation that arises. For example, if one unobserved variable here the haplotype frequency has a relatively small value and another has a relatively large value, then one could possibly set wider limits for the equations of smaller value to deem when they are in equilibrium.

It will be appreciated that the definition of equilibrium can vary depending on the specific study being carried out. Further, this will vary when the technique is used for different technologies.

Further, it is envisaged that likelihood equations will be used such than when a preset number of equations have reached equilibrium, the value of the equations are inserted into a likelihood equation and the values are then stored. Further random numbers are used to redo the equations and then a further set of equations will be solved which will reach equilibrium and again the likelihood equation is again used and the result of the likelihood will transfer to the memory. After a sufficient number of sets of linear equations have been solved, the likelihood equation results are identified and the most likely solution is then taken. It is further envisaged that with various equations, the likelihood equation may be used to determine when equilibrium is reached.

Coupled nonlinear equations frequently turn up in as a result of applying the technique of maximum likelihood estimation, which is central to the EM algorithm. In genetics this not only includes testing for linkage disequilibrium, but also haplotype frequency estimation (HFE).

Further in one application the invention provides a computerised system for the genetic analysis of genotype data to discover the gene or genes responsible for a particular trait or by predicting the existence of linage disequilibrium (LD) between two loci using an expectation-maximisation (EM) algorithm giving rise to coupled non-linear equations, the computerised system comprising an input device, a memory and a central processing unit (CPU) connected to the input device to receive genetic data therefrom and for the delivery to and retrieval from the memory of data, the CPU being programmed to carry out iterations of the computation of the non-linear equations, characterised in that the system further comprises:-

detection means for ascertaining when the equation has reached an equilibrium defined by determining that the term or result provided by the equation has changed by an amount less than a predetermined amount;

removal means to extract the equation from the CPU and transfer it to memory for storage on equilibrium being reached;

re-insertion means to retrieve, after a preset further number of iterations of the equation, the equation from the memory and to transfer the equation to the CPU for further iterations of the computation whereby the computational requirements and thus the analysis time of the system is reduced.

This latter computerised system for the genetic analysis of geno-type data may have removal means, re-insertion means, detection means similar to those described above.

Further, in essence the invention provides a method for solving coupled non-linear equations comprising:-

carrying out an iteration of the set of non-linear equations;

determining when an equation approaches equilibrium;

removing the equation from the set to provide a reduced size set of equations;

carrying out a preset number of iterations of the reduced size set of equations;

replacing the equation in the reduced size set to form a new set of equations; and

carrying out a further iteration of the new set of the equations.

In accordance with the method when after a preset number of iterations of the computations subsequent to re-insertion, the equation is in equilibrium the equation is again removed from the set of equations and the computation is carried out for a further preset number of iterations of the equations without the removed equation.

The method further provides that if on re-insertion of the equation the value of the equation is in equilibrium with the value of the equation prior to removal, the equation is again removed for the preset further iterations.

In accordance with the method, more than one equation may be removed at the one time or indeed at successive iterations an equation may be removed. Ideally an equation is removed for between two and five iterations of the equation but may be generally about five times. Again this may be changed and some equations will be removed for longer periods than others depending on their relevance to the overall result required.

Equilibrium is defined as being reached when the value of the equation for successive iterations varies by less than a preset percentage of the value of one or other of the two values. This will often be a percentage of between $10^{-2}$ and $10^{-4}$.

In accordance with the method of the invention, when a preset number of equations have reached equilibrium, the value of the equations are inserted into a likelihood equation and further iteration of the equations is stopped if the result of the likelihood equation is such as to indicate that further iterations are unlikely to produce a result, the likelihood of which would be possible. This needless to say would depend on the particular technology to which the equation is being applied.

In one method of carrying out the invention when at least some of the equations have reached equilibrium, the values of the equations are inserted into a likelihood equation and the result of the likelihood equation is stored for future reference. Ideally this is carried out when all the equations have reached equilibrium. The method may be applied to the conducting of gene hunting studies in which case when some of the values of the equations equal 1 and the equations would be deemed to be in equilibrium when the value of the equations for successive iterations varied by less than $10^{-6}$.

The method as described may be used to analyse linkage disequilibrium (LD) namely deviation from random occurrence of alleles in haplotypes by using an expectation-maximisation (EM) algorithm to produce the equations and the system includes means to assign values for the haplotype frequencies of the haplotypes of two alleles one from each of two loci. Preferably in this method values will be assigned to two loci. These may be random values, the aggregate of which is one.

The method according to the present invention may be carried out by a computer and the means may be instructive to a computer as a computer program when embodied on a record medium a computer program, a read-only memory or carried on an electrical signal carrier.

The freezing/unfreezing technique is applicable to all cases of coupled nonlinear equations, though it is more efficient the greater number of equations to be solved. In the cases of LD and HFE, where all haplotype frequencies must be non-negative and sum to one, the stopping conditions (when equilibrium is said to have been achieved) are when the average change across the total set of frequencies drops below a certain constant value for one iteration. When no such constraints on the size and sign of the variables to be solved for exist, then a possible equilibrium condition is that the variables change by less than a set percentage from one iteration to the next. As the EM algorithm results in a likelihood (here given by L), which in the case of LD is derived from the haplotype frequencies, equilibrium may also be said to have been reached when the change in the likelihood falls below a certain set percentage from one iteration to the next. The percentage would vary from problem to problem, depending on the desired accuracy of the user.

The necessity of solving coupled non-linear equations occurs in many technologies where the analysis of complex variable data arises. While the present specification describes the use of the computerised system with particular reference to genetic data and gene hunting studies, it has obvious wider applications, particularly in materials and structural design analysis production control and other operations. The industrial applications are endless. Indeed, it is envisaged that in many other technologies, greater savings in computational time may be achieved, particularly where equilibrium is more quickly achieved or the effects of the value of a specific equation are likely to be less significant than that of another.

In the specification the terms "comprise, comprises, comprised and comprising" or any variation thereof and the terms "include, includes, included and including" or any variation thereof are considered to be totally interchangeable and they should all be afforded the widest possible interpretation.

The invention is not limited to the embodiments hereinbefore described but may be varied in both construction and detail within the scope of the claims.

**Claims**

1. A computerised system (1) for the solving of coupled non-linear equations having a plurality of terms of the type comprising:-

   an input device (2);

   a memory (3);

   a central processing unit (CPU) (4) connected to the input device (2) and the memory (3) for carrying out iterations of the non-linear equations for varying values of each term of the equation **characterised in that** there is provided detection means (6) for ascertaining when an equation has reached equilibrium after a plurality of computation iterations of the equations by the CPU (4);

   removal means (7) to extract the equation from the CPU (4) when the equation has reached equilibrium and to transfer the equation to the memory (3) for storage; and

   re-insertion means (8) to retrieve the equation after a preset further number of iterations of the equation and to transfer the equation to the CPU (4) for further iterations of the computation thereby reducing the computational time for solving the equation.

2. A system (1) as claimed in claim 1, in which the removal means (7) is arranged so that, if after a preset number of iterations of the computation subsequent to re-insertion, the equation is in equilibrium, the removal means (7) again transfers the equation to the memory (3) from the computation.

3. A system (1) as claimed in claim 2, in which if after re-insertion of the equation, the removal means (7) is arranged so that if the value of the equation is in equilibrium with the value of the equation prior to removal, the equation is again transferred to the memory (3) by the removal means (7) for the preset further iterations.

4. A system (1) as claimed in any preceding claim, in which the removal means (7) is arranged so that more than one equation may be transferred to the memory (3) at any one time.

5. A system (1) as claimed in any preceding claim, in which the re-insertion means (8) is arranged so that the equation is re-inserted after between two and ten iterations.

6. A system (1) as claimed in any of claims 1 to 4 in which the re-insertion means (8) is arranged so that the equation is re-inserted after five iterations.

7. A system (1) as claimed in any preceding claim, in which the detection means (6) is adapted to determine that equilibrium is reached when it senses that the value of the equation for successive iterations varies by less than a preset percentage of the value of one or other of the two values.

8. A system (1) as claimed in claim 7, in which the percentage is between $10^{-2}$ and $10^{-4}$.

9. A system (1) as claimed in any preceding claim, in which the detection means (6) is arranged so that when a preset number of equations have reached equilibrium, the values of the equations are inserted into a likelihood equation and termination means (9) are provided for stopping further iterations of the equations if the result of the likelihood equation is such as to indicate that further iterations are unlikely to produce a result, the likelihood of which would be possible.

10. A system (1) as claimed in any of claims 1 to 8, in which when at least some of the equations have reached equilibrium, the detection means (6) is arranged to insert the values into a likelihood equation and the results of the likelihood equation are transferred to the memory (3).

11. A system (1) as claimed in claim 10, in which the operation is carried out when all the equations have reached equilibrium.

12. A system (1) as claimed in any preceding claim, in which when conducting a gene hunting study the sum of the value of the equations equals one and the equations are deemed to be in equilibrium when the value of the equations for successive iteration varies by less than $10^{-6}$.

13. A system (1) as claimed in any preceding claim, in which the system is used to analyse linkage disequilibrium (LD) namely deviation from random occurrence of alleles in haplotypes by using an expectation-maximisation (EM) algorithm to produce the equations and the system (1) includes means to assign values for the haplotype frequencies of the haplotypes of two alleles one from each of two loci.

14. A system (1) as claimed in claim 13, in which the means to assign values includes means to assign values to two loci.

15. A system (1) as claimed in claim 13 or 14, in which the means to assign values includes means to assign random values, the aggregate of which is one.

16. A computer program comprising program instructions which, when loaded into a computer, constitute the means of any of claims 1 to 15.

17. A computer program as claimed in claim 16, embodied on a record medium.

18. A computer program as claimed in claim 16, stored in a computer memory.

19. A computer program as claimed in claim 16, embodied in a read-only memory.

20. A computer program as claimed in claim 16, carried on an electrical signal carrier.

Fig. 1

**Fig.2**

Fig. 3

EP 1 333 383 A1

Initialise frequencies

Move to first equation

**New Technology**

Equation frozen ($F_{ij}$>0)?

No → Calculate and implement change. Has it reached equilibrium?

Yes → Freeze equation. $F_{ij}$ = G.

Yes ↓

Reduce $F_{ij}$ by 1

No ↓

Move to next equation

No

All equations completed?

Yes → Total change < tolerance? → No

Yes ↓

End computation

**Fig.4**

## EUROPEAN SEARCH REPORT

**European Patent Office**

| | Application Number |
|---|---|
| | EP 02 39 4017 |

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | BONNEN P E ET AL: "Haplotypes at ATM identify coding-sequence variation and indicate a region of extensive linkage disequilibrium." AMERICAN JOURNAL OF HUMAN GENETICS. UNITED STATES DEC 2000, vol. 67, no. 6, December 2000 (2000-12), pages 1437-1451, XP002210145 ISSN: 0002-9297 * page 1437, left-hand column, line 16 – page 1438, left-hand column, line 45 * * page 1438, right-hand column, line 34 – page 1439, right-hand column, line 42 * * page 1440, left-hand column, line 17 – page 1443, left-hand column, line 19 * * page 1446, left-hand column, line 32 – page 1448, right-hand column, line 39 * --- | 1-20 | G06F17/12 G06F19/00 |
| A | FALLIN D ET AL: "Accuracy of haplotype frequency estimation for biallelic loci, via the expectation-maximization algorithm for unphased diploid genotype data." AMERICAN JOURNAL OF HUMAN GENETICS. UNITED STATES OCT 2000, vol. 67, no. 4, October 2000 (2000-10), pages 947-959, XP002210146 ISSN: 0002-9297 * page 948, left-hand column, line 27 – page 952, right-hand column, line 4 * * page 953, right-hand column, line 4 – line 18 * * page 954, left-hand column, line 10 – page 956, right-hand column, line 3 * --- -/-- | 1-20 | TECHNICAL FIELDS SEARCHED (Int.Cl.7) G06F |

The present search report has been drawn up for all claims

| Place of search | Date of completion of the search | Examiner |
|---|---|---|
| MUNICH | 16 August 2002 | Barba, M |

EPO FORM 1503 03.82 (P04C01)

**European Patent Office**

**EUROPEAN SEARCH REPORT**

Application Number

EP 02 39 4017

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WEINBERG C R: "Allowing for missing parents in genetic studies of case-parent triads." AMERICAN JOURNAL OF HUMAN GENETICS. UNITED STATES APR 1999, vol. 64, no. 4, April 1999 (1999-04), pages 1186-1193, XP002210147 ISSN: 0002-9297 * page 1186, left-hand column, line 1 - line 26 * * page 1188, left-hand column, line 34 - page 1189, right-hand column, line 18 * * page 1191, left-hand column, line 8 - right-hand column, line 24 * | 1-20 | |
| A | WEINBERG C R ET AL: "A log-linear approach to case-parent-triad data: assessing effects of disease genes that act either directly or through maternal effects and that may be subject to parental imprinting." AMERICAN JOURNAL OF HUMAN GENETICS. UNITED STATES APR 1998, vol. 62, no. 4, April 1998 (1998-04), pages 969-978, XP002210148 ISSN: 0002-9297 * page 969, left-hand column, line 1 - line 29 * * page 970, right-hand column, line 16 - page 971, right-hand column, line 10 * * page 972, right-hand column, line 9 - page 973, right-hand column, line 5 * * page 974, right-hand column, line 23 - page 975, left-hand column, line 20 * ---   -/-- | 1-20 | TECHNICAL FIELDS SEARCHED (Int.Cl.7) |

The present search report has been drawn up for all claims

| Place of search | Date of completion of the search | Examiner |
|---|---|---|
| MUNICH | 16 August 2002 | Barba, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 02 39 4017

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | UEDA N ET AL: "Split and merge EM algorithm for improving Gaussian mixture density estimates" NEURAL NETWORKS FOR SIGNAL PROCESSING VIII, 1998. PROCEEDINGS OF THE 1998 IEEE SIGNAL PROCESSING SOCIETY WORKSHOP CAMBRIDGE, UK 31 AUG.-2 SEPT. 1998, NEW YORK, NY, USA,IEEE, US, 31 August 1998 (1998-08-31), pages 274-283, XP010298236 ISBN: 0-7803-5060-X * page 276, line 10 - page 278, line 6 * * page 278, line 13 - page 279, line 4 * ----- | 1-20 | |
| | | | **TECHNICAL FIELDS SEARCHED (Int.Cl.7)** |

The present search report has been drawn up for all claims

| Place of search | Date of completion of the search | Examiner |
|---|---|---|
| MUNICH | 16 August 2002 | Barba, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)